(19) Europäisches Patentamt
European Patent Office
Office européen des brevets

(11) Publication number: **0 145 177**
**B1**

(12) **EUROPEAN PATENT SPECIFICATION**

(45) Date of publication of patent specification: **22.02.89**

(21) Application number: **84307118.4**

(22) Date of filing: **17.10.84**

(51) Int. Cl.$^4$: **A 61 K 31/42** // C07D493/22, (C07D493/22, 313:00, 311:00, 303:00, 303:00)

(54) **Chemotherapeutic agents based on rhizoxin.**

(30) Priority: **17.10.83 JP 193840/83**

(43) Date of publication of application:
**19.06.85 Bulletin 85/25**

(45) Publication of the grant of the patent:
**22.02.89 Bulletin 89/08**

(84) Designated Contracting States:
**BE CH DE FR GB IT LI NL SE**

(56) References cited:
**CHEMICAL ABSTRACTS, vol. 101, no. 7, 13 August 1984, Columbus, Ohio (US); S.IWASAKI et al.: "Studies on macrocyclic lactone antibiotics. VII. Structure of a phytotoxin 'rhizoxin' produced by Rhizopus chinensis", p. 309, no. 51352c**

(73) Proprietor: **National Institut of Agro-Environmental Sciences, Ministry of Agriculture, Forestry, and Fisheries**
**3-1-1, Kannondai Yatabe-machi**
**Tsukuba-gun Ibaraki-ken (JP)**

(72) Inventor: **Sato, Zenji**
**3-627, Namiki Sakura-mura**
**Niihari-gun Ibaraki-ken (JP)**
Inventor: **Matsuda, Izumi**
**9-12-1, Togashira**
**Toride-shi Ibaraki-ken (JP)**
Inventor: **Noda, Takahito**
**1-4-14, Inada**
**Jouetsu-shi Niigata-ken (JP)**
Inventor: **Okuda, Shigenobu**
**8-23-10, Shakujii-cho**
**Nerima-ku Tokyo (JP)**
Inventor: **Iwasaki, Shigeo**
**2-31-12-402, Nishi-ikebukuro**
**Toshima-Ku Tokyo (JP)**
Inventor: **Furukawa, Jun**
**4-20-4, Kajino-cho**
**Koganei-shi Tokyo (JP)**
Inventor: **Kobayashi, Hisayoshi**
**7-15-2-108, Shimo-renjaku**
**Mitaka-shi, Tokyo (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 145 177 B1**

Courier Press, Leamington Spa, England.

(74) Representative: **Ablewhite, Alan James et al**
**MARKS & CLERK 57/60 Lincoln's Inn Fields**
**London WC2A 3LS (GB)**

## Description

This invention relates to compositions of use in chemotherapy, particularly of tumors of human beings and other mammals, containing Rhizoxin which is a metabolic product of the widespread plant pathogenic fungus *Rhizopus chinensis*.

In recent years, the wide spread of the rice culture using mechanical transplanting methods has led to the spread of a variety of diseases on seedlings raised in nursery boxes. In particular, the damage by the seedling blight caused by *Rhizopus chinensis* is above all outstanding. The seedlings infected by this pathogen develop some characteristic symptoms such that the growth is considerably checked in both the parts above and under the ground; the root apex and the basal part of the coleoptile become swollen and so on. Since it was presumed that a certain substance produced by the pathogenic fungus present might be responsible for the development of such symptoms, the present inventors carried out the isolation and purification of that substance and succeeded in obtaining a novel substance, which was named "Rhizoxin".

Rhizoxin can be obtained by culturing *Rhizopus chinensis* (any wild strain) in the routine manner and then after extracting the culture filtrate by carrying out the isolation and purification.

The physical and chemical properties of Rhizoxin obtained in this way are shown as follows.

| | |
|---|---|
| External appearance: | White powder |
| Molecular formula: | $C_{35}H_{47}NO_9$ |
| Molecular weight: | 625 |
| Melting point: | 131—135°C |
| Specific rotation: | $[\alpha]^{24}$ + 201° (c=0.8, MeOH) |
| Ultraviolet absorption spectrum: | Shown in Fig. 1. |
| Infrared absorption spectrum: | Shown in Fig. 2, in which chloroform is used as the solvent. |
| Nuclear magnetic resonance spectrum: | $^1H$ and $^{13}C$ nuclear magnetic resonance spectra are shown in Fig. 3. |
| Mass spectrum: | Shown in Fig. 4. |
| Solubility in solvents: | Easily soluble in benzene, chloroform, acetone, alcohol, etc.; sparingly soluble in water, n-hexane. |

Elementary analysis:

| | C | H | N |
|---|---|---|---|
| Found (%): | 66.94 | 7.78 | 2.04 |
| Calculated (%): | 67.18 | 7.57 | 2.23 |

As a result of summarizing all these data it was concluded that Rhizoxin has the following skeletal structure.

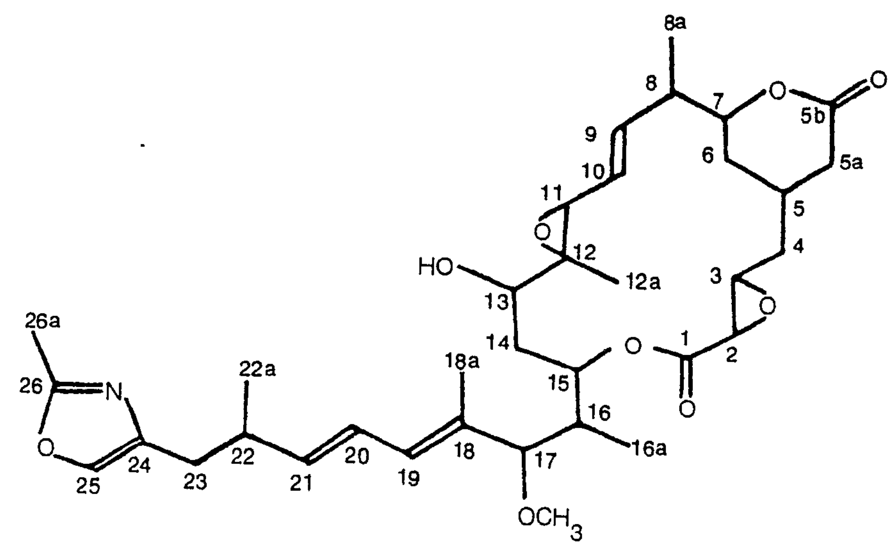

In this specification, however, reference to Rhizoxin should be taken to refer to the substance extractable from *Rhizopus chinensis* and having the stated physical properties.

Rhizoxin has an action which can induce the characteristic symptoms of rice seedling blight, even at an extremely low concentration (10 ng/ml). Besides the above, not only does it check the growth of the root of

the plants other than rice, but also inhibits the growth of plant pathogenic fungi. However, it has no recognizable action on the multiplication of the plant pathogenic bacteria that are porokaryotes.

The above described facts were presented in the 25th Symposium on the Chemistry of Natural Products held in Tokyo on October 19—22, 1982 (Collection of Synopses of Papers Read, p. 491), by the inventors of the present invention after which the biological activity of Rhizoxin was further studied by the present inventors, and it has surprisingly now been discovered that said compound has a remarkable antitumor activity.

It is one object of the invention of this application to provide a pharmaceutical composition, especially for palliative relief and temporary remission of tumor on human beings and other mammals, which comprises Rhizoxin in an effective amount to exert said relief and remission and a pharmaceutically acceptable, non-toxic carrier.

It has been confirmed that Rhizoxin has a strong antitumor activity against MH-134 cells due to cancer of the liver transplanted to mice; L-1210 cells due to leukemia; P-388 cells due to the same; as well as P-388 cells resistant to vincristine, a carinostatic agent now in wide use; B-16 melanoma, etc.

With reference to several test examples, the antitumor activity of Rhizoxin will be explained below.

Test Example 1

$1 \times 10^6$ of ascites tumor MH-134 cells due to the cancer of the liver were intraperitoneally inoculated in to C3H/He mice (8 weeks old, each group 6 mice). Rhizoxin was dissolved with the addition of a small quantity of ethyl alcohol (not more than 1 volume percent on the basis of the total volume of the final solution), and further dissolved in a 0.85% aqueous physiological sodium chloride solution containing 0.05% of Tween 20. This solution was aseptically filtered, and 24 hours after the tumor inoculation, the solution was intraperitoneally administered to the mice continually for 10 days at a rate of 0.2 ml/mouse once a day. The antitumor activity of Rhizoxin was evaluated on the basis of the ratio $(T/C) \times 100$ wherein T is the average life span of the Rhizoxin administered groups and C is the average life span of the control group. The result is shown in Table 1.

TABLE 1

| Dose (mcg/Kg/day) | Number of mice tested | Average life span (days) | T/C (%) |
|---|---|---|---|
| 150 | 6 | 47.8 | 278 |
| 15 | 6 | 31.8 | 185 |
| Control | 6 | 17.2 | — |

Test Example 2

Leukemia L-1210 cells ($1 \times 10^5$ cells) were intraperitoneally inoculated into $CDF_1$ mice. Intraperitoneal administration of Rhizoxin was started 24 hours after the tumor inoculation and continued daily for 4 days. The result is shown in Table 2 in terms of a percentage of an increase in the life span of the treated mice over that of the control mice (ILS).

TABLE 2

| Dose (mg/Kg/day) | Change in body weight (g) (day 1—7) | ILS (%) |
|---|---|---|
| 0.25 | +0.4 | 43 |
| 0.5 | −0.7 | 67 |
| 1.0 | −2.9 | 81 |
| 2.0 | | toxic |

Test Example 3

Tumor fragments of B-16 melanoma were subcutaneously inoculated into $BDF_1$ mice. Intraperitoneal administration of Rhizoxin was begun 24 hours after the tumor inoculation and continued daily for 4 days. The result is shown in Table 3 in terms of a percentage of tumor growth inhibition based on the tumor weight which can be obtained from the measurement of the tumor diameter.

TABLE 3

| Dose (mg/Kg/day) | Tumor growth inhibition (%) |
|---|---|
| 0.25 | 38 |
| 0.5 | 68 |
| 1.0 | 62 |

Test Example 4

Vincristine resistant or sensitive leukemia P-388 cells ($1 \times 10^6$ cells) were intraperitoneally inoculated into $CDF_1$ mice. Intraperitoneal administration of Rhizoxin and Vincristine was made on 1, 6 and 9 days after the tumor inoculation. The result is shown in Table 4 in terms of a percentage of an increase in the average life span of the treated mice over that of the control mice.

As easily understood from the table, Rhizoxin is effective against leukemia P-388, and moreover, it is also clearly effective against Vincristine resistant leukemia P-388 which was not affected by Vincristine.

TABLE 4

| Tumor inoculated | Agent administered | Dose (mg/Kg/day) | ILS (%) |
|---|---|---|---|
| P-388 | Rhizoxin | 0.5 | 46.2 |
| | | 0.75 | 65.6 |
| | | 1.0 | 82.9 |
| | | 1.5 | 123.7 |
| | | 3.0 | toxic |
| P-388/VCR | Rhizoxin | 0.75 | 28.2 |
| | | 1.0 | 35.9 |
| | | 1.5 | 57.3 |
| | | 2.0 | 61.2 |
| | | 4.0 | toxic |
| | Vincristine | 0.75 | −6.8 |
| | | 1.0 | 6.8 |
| | | 2.0 | toxic |

The toxicity of Rhizoxin was examined by the intraperitoneal injection of said compound into C3H/He mice, and as the $Ld_{50}$ value was above 5 mg/kg, it may be mentioned that the value is far above the dosage usually given in treatment.

From the foregoing results it will be readily recognized by those skilled in the art that Rhizoxin is useful as the antitumor agent to human beings and other mammals. As the form of administration there may be mentioned parenteral administration such as subcutaneous injection, intravenous injection, intramuscular injection, or the like, as well as oral administration. The dosage to be given to an adult, is usually in the range of 0.1—20 mg per day, though varying depending on the subject disease, the route of administration, and the number of times of administration; for instance, 0.3—2 mg is dosed making a division into one or more of single doses. Rhizoxin may also be used in combination with other antitumor agents such as nitrosoureas, e.g., ACNU, BCNU, etc.; Cisplatinum, 5-fluorouracil, daunomycin, adriamycin, mitomycin, etoposide, etc.

Rhizoxin may also be prepared in the unit dosage form by any conventional method. This invention, therefore, embraces further the Rhizoxin-containing preparation and composition that are pharmaceutically suitable. The injectable composition is provided in ampoules containing unit dosage or vessels containing multiple dosages. The composition may often comprise Rhizoxin singly, but various additives such as suspending agent, stabilizer, and/or dispersing agent may also be added thereto. Usually it is provided in the form of a powder which can be dissolved or suspended in a suitable solvent such as, for instance, a sterile aqueous medium containing no exothermic material before use. The preparation of this sort can be prepared, for instance, in such a manner that after Rhizoxin has been dissolved in acetone, the solution is divided into small portions, and each portion is pipetted into a vial and then freeze-dried with the addition of water. As the solvent used for dissolution or suspension of this preparation use can be made of any conventional one, such as, for instance, an organic solvent, e.g., ethyl alcohol, benzyl alcohol, Polyethylene glycol, N,N-dimethylacetamide, in which case it is preferable that an aqueous medium containing anionic surfactant, sodium chloride, glucose, etc is added to the solution or suspension thus obtained. In addition to the above, the oral composition is provided in the form of tablets, capsules, powders, granules, syrups, etc. These preparations are prepared by any conventional method using a conventional vehicle such as, for instance, lactose, Avicel®, mannitol, starch, sucrose, magnesium stearate, talc, etc. Coating agents, sweetenings, corrigents, flavorings, aromatics, etc may also be used as the occasion may demand.

With reference to the following examples this invention will be more concretely explained, but it should be understood that this invention should not be limited thereto.

Reference Example 1

Stationary culture of Rhizoxin-producing fungus, *Rhizopus chinensis,* was made in a culture medium containing glucose (1%), polypepton (1%), magnesium sulfate heptahydrate ($MgSO_4 \cdot 7H_2O$)(0.05%), potassium biphosphate ($KH_2PO_4$)(0.05%), dipotassium phosphate ($K_2HPO_4$)(0.05%), and calcium chloride dihydrate ($CaCl_2 \cdot 2H_2O$)(0.01%) for 2 days at 32°C in Petri dishes.

The culture filtrate was extracted with diethyl ether, and after the ether has been evaporated, the residue was suspended in water and n-hexane-soluble matter was removed. Next, the n-hexane-insoluble residue was extracted with benzene, and after the benzene had been evaporated, the final residue was purified by silica gel chromatography to give crude Rhizoxin crystals.

The crude Rhizoxin was further purified by Sephadex LH-20 column and silica gel column to give pure Rhizoxin crystals. The yield of the crude Rhizoxin was about 2 mg/l culture, and the yield of the pure Rhizoxin was about 1.3 mg/l culture.

Reference Example 2

Shaking culture of *Rhizoxin chinensis* was made in a culture medium containing glucose (1%), polypeptone (1%), potassium biphosphate ($KH_2PO_4$)(0.05%), dipotassium phosphate ($K_2HPO_4$)(0.05%), and magnesium sulfate heptahydrate ($MgSO_4 \cdot 7H_2H$)(0.25%) for 96 hours at 30°C.

Then, the culture filtrate was purified by quite the same procedure as in Reference Example 1 to give pure Rhizoxin crystals. The yields of the crude and pure Rhizoxin were respectively about one half of the yield in the case of the stationary culture in Reference Example 1.

Example 1

60 mg of Rhizoxin is dissolved in 20 ml of acetone, and 180 ml of sterile distilled water is added thereto. 1 ml each of suspension of the above Rhizoxin is aseptically pipetted into a vial having a capacity of 3 ml, and by freeze-drying there is obtained an injection of the type readily soluble in use, which contains 0.3 mg of Rhizoxin per vial.

Example 2

60 mg of Rhizoxin is dissolved in 12 ml of acetone and 0.1 mg each of the solution is aseptically pipetted into a vial, 3—5 ml in capacity, containing 1 ml of sterile distilled water, and by freeze-drying there is obtained an injection of the type readily soluble in use, which contains 0.5 mg of Rhizoxin per vial.

Example 3

979 parts by weight of lactose is added to 10 parts by weight of Rhizoxin, 10 parts by weight of L-HPC, and 1 part by weight of magnesium stearate, and after thorough grinding and mixing the resulting product is compressed into tablets in the routine manner so that each tablet has a weight of 100 mg. Thus, there are obtained oral tablets.

**Claims**

1. A pharmaceutical composition for administration to human beings and other mammals which comprises Rhizoxin and a pharmaceutically acceptable, non-toxic carrier.

2. A pharmaceutical composition according to claim 1 wherein said composition is in the unit dosage form for oral or parenteral administration.

3. A pharmaceutical composition according to claim 1 wherein said composition is in the form of tablets, capsules, powders, granules, or syrup.

4. A pharmaceutical composition according to claim 1 wherein said composition is in the form of injectable solutions or suspensions.

5. A pharmaceutical composition according to claim 1 wherein said composition is in the unit dosage form of a powder capable of being dissolved or suspended in a solvent system immediately before administration.

6. A pharmaceutical composition according to claim 5 wherein said solvent system is composed of ethanol, benzyl alcohol, N,N-dimethylacetamide or polyethylene glycol and an aqueous medium containing nonionic surfactant, sodium chloride, or glucose.

7. A pharmaceutical composition according to claim 5 wherein said unit dosage form is prepared by dissolving Rhizoxin in acetone, adding the resulting solution to sterile water for injection and then freeze-drying.

8. A composition according to any of claims 1 to 8 at a concentration of Rhizoxin sufficient for a daily dose of 0.1 mg to 20 mg to be administered conveniently.

9. A composition according to claim 8 formulated to provide a single dose of 0.3 mg to 2 mg of Rhizoxin.

10. Rhizoxin for use as a chemotherapeutic agent in humans and other mammals.

**Patentansprüche**

1. Pharmazeutisches Mittel zur Verabreichung an Menschen und (andere) Säugetiere, welches Rhizoxin und einen pharmazeutisch akzeptablen ungiftigen Träger umfaßt.

2. Pharmazeutisches Mittel nach Anspruch 1, welches in Form einer Dosierungseinheit zur oralen oder parenteralen Verabreichung vorliegt.

3. Pharmazeutisches Mittel nach Anspruch 1, welches in Form von Tabletten, Kapseln oder Pulvern oder als Granulat oder Sirup vorleigt.

4. Pharmazeutisches Mittel nach Anspruch 1, welches in Form von injizierbaren Lösungen oder Suspensionen vorliegt.

5. Pharmazeutisches Mittel nach Anspruch 1, welches in Form einer Dosierungseinheit eines Pulvers vorliegt, welches unmittelbar vor der Verabreichung in einem Lösungsmittelsystem auflösbar oder suspendierbar ist.

6. Pharmazeutisches Mittel nach Anspruch 5, bei dem das Lösungsmittelsystem aus Ethanol, Benzylalkohol, N,N-Dimethylacetamid oder Polyethylenglykol und einem wässrigen Medium zusammengesetzt ist, welches ein nicht-ionisches Netzmittel, Natriumchlorid oder Glukose, enthält.

7. Pharmazeutisches Mittel nach Anspruch 5, bei dem die Form der Dosierungseinheit bereitet wird, indem man Rhizoxin in Aceton auflöst und die dabei erhaltene Lösung sterilem Wasser zum Injizieren zusetzt und dann gefriertrocknet.

8. Mittel nach einem der Ansprüche 1 bis 7 mit einer Konzentration von Rhizoxin, die für eine bequem zu verabreichende tägliche Dosis von 0,1 mg bis 20 mg ausreicht.

9. Mittel nach Anspruch 8, wleches so zusammengesetzt ist, daß sich eine einzige Dosis von 0,3 mg bis 2 mg Rhizoxin ergibt.

10. Rhizoxin zur Verwendung als chemotherapeutischer Wirkstoff für Menschen und (andere) Säugetiere.

**Revendications**

1. Une composition pharmaceutique administrable à des êtres humains ou à d'autres mammifères qui comprend de la Rhizoxine, et un support non toxique acceptable en pharmacie.

2. Une composition pharmaceutique selon la revendication 1 dans laquelle ladite composition est sous forme de dose unitaire pour administration orale ou parentérale.

3. Une composition pharmaceutique selon la revendication 1 dans laquelle ladite composition est sous forme de comprimés, capsules, poudres, granulés, ou sirop.

4. Une composition pharmaceutique selon la revendication 1 dans laquelle ladite composition est sous forme de solutions ou suspensions injectables.

5. Une composition pharmaceutique selon la revendication 1 dans laquelle ladite composition sous forme de dose unitaire est une poudre capable d'être dissoute ou mise en suspension dans un solvant immédiatement avant administration.

6. Une composition pharmaceutique selon la revendication 5 dans laquelle ledit solvant est composé d'éthanol, d'alcool benzylique, de N,N-diméthylacétamide ou de polyéthylène glycol et d'un milieu aqueux contenant un agent tensio-actif non ionique, du chlorure de sodium ou du glucose.

7. Une composition pharmaceutique selon la revendication 5 où la dose unitaire est préparée par dissolution de la Rhizoxine dans de l'acétone, et addition de la solution ainsi obtenue à de l'eau stérile pour préparation injectable puis lyophilisée.

8. La composition pharmaceutique selon les revendications 1 à 7 à une concentration de Rhizoxine suffisante pour une dose quotidienne de 0,1 mg à 20 mg à administrer sans inconvénient.

9. Une composition selon la revendication 8 formulée pour fournir une dose de 0,3 mg à 2 mg de Rhizoxine.

10. La Rhizoxine pour un usage en tant qu'agent chimiothérapeutique chez l'homme et d'autres mammifères.

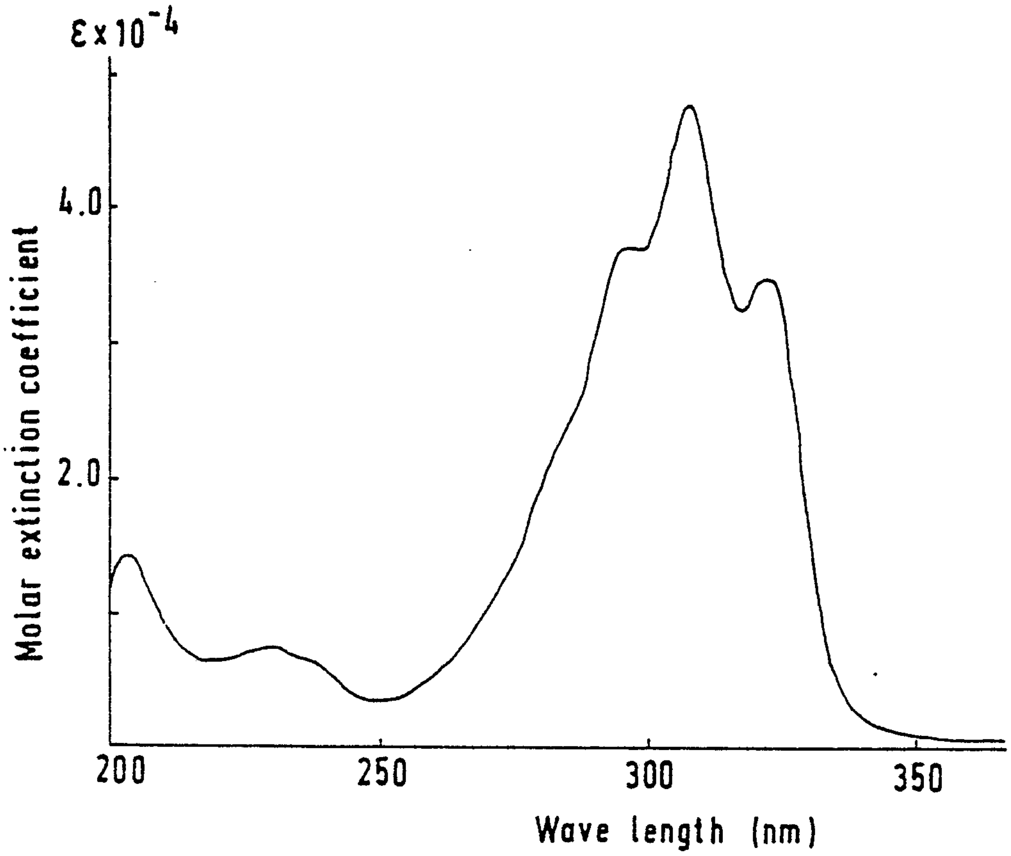
ε x 10^-4
4.0
2.0
Molar extinction coefficient
200
250
300
350
Wave length (nm)

FIG.1.

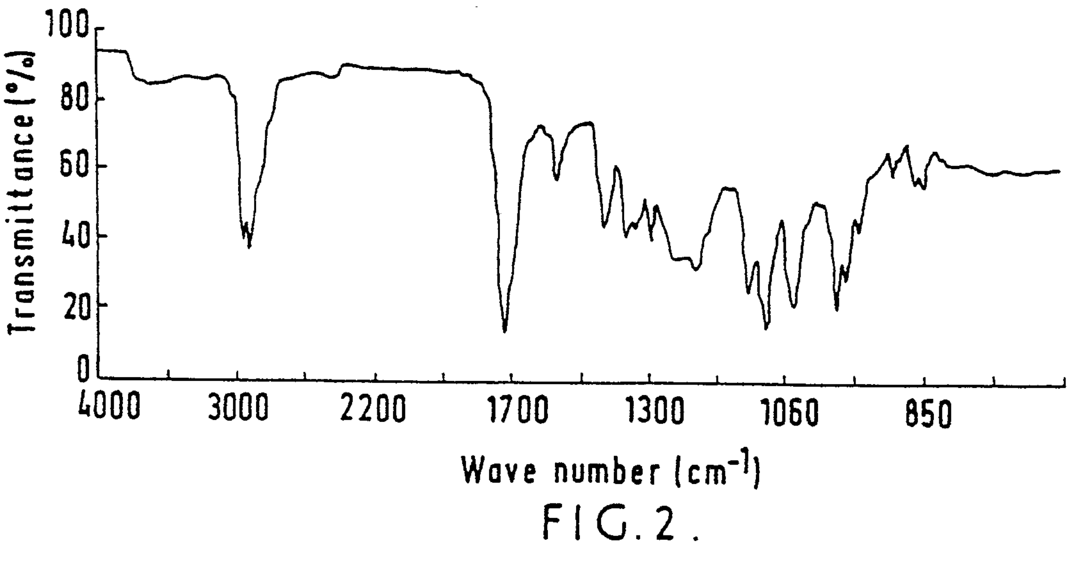
100
80
60
40
20
0
Transmittance (%)
4000
3000
2200
1700
1300
1060
850
Wave number (cm^-1)

FIG.2.

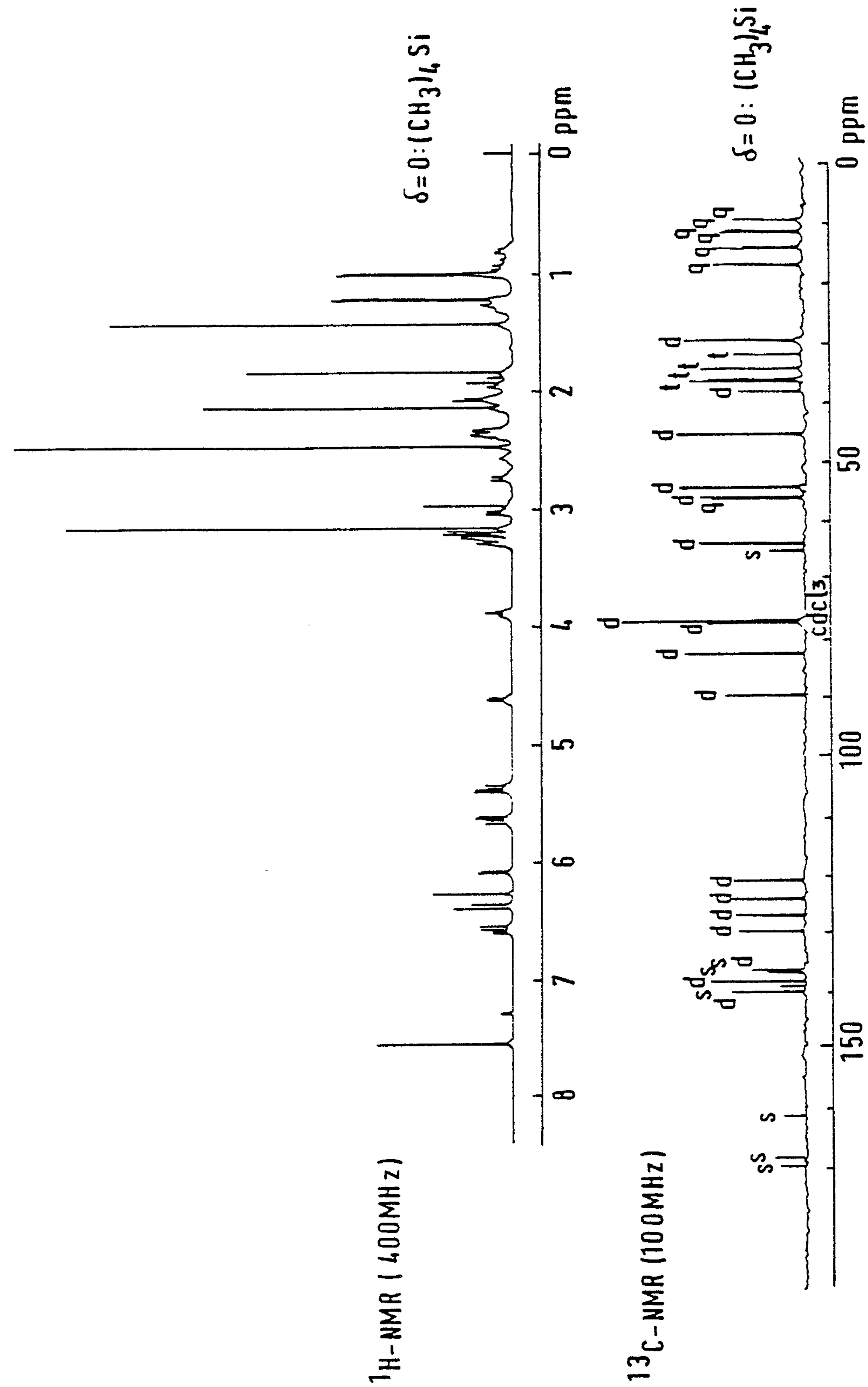

1H-NMR (400MHz)
13C-NMR (100MHz)
δ=0:(CH3)4Si
δ=0: (CH3)4Si
0 ppm
0 ppm
CDCl3


FIG. 3.

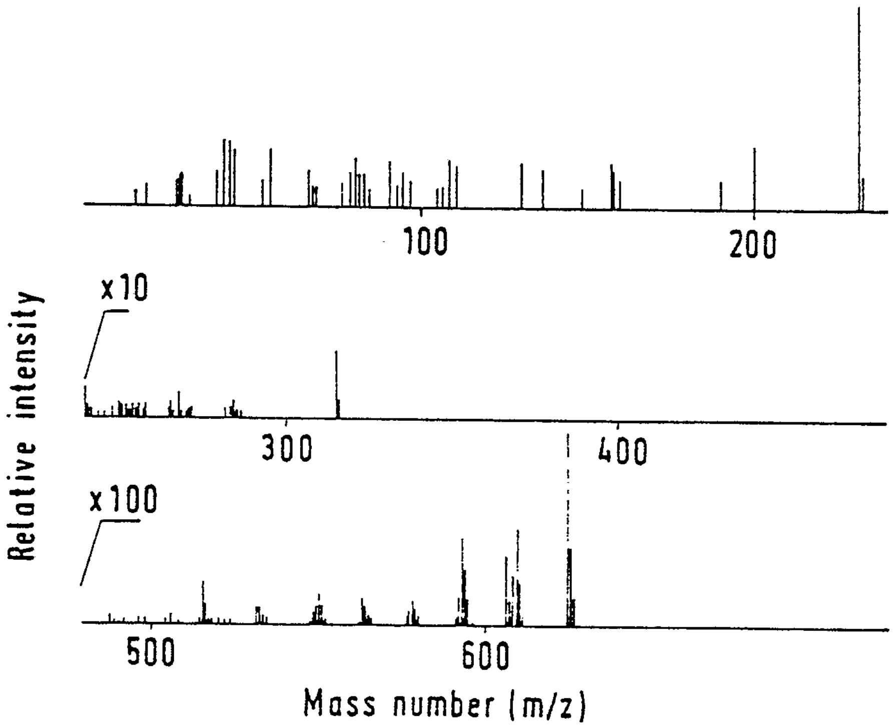
100
200
x10
Relative intensity
300
400
x100
500
600
Mass number (m/z)

FIG. 4